# EUROPÄISCHE PATENTSCHRIFT

(11) **EP 1 140 259 B1**
(45) Veröffentlichungstag und Bekanntmachung des Hinweises auf die Patenterteilung: **16.03.2005**
(21) Anmeldenummer: 00971406.4
(22) Anmeldetag: 02.11.2000
(51) Int. Cl.: A61M 5/19

(54) **MEHRKAMMER-AMPULLE ZUM AUSGEBEN EINES AUS MEHREREN SUBSTANZEN BESTEHENDEN GEMISCHES**
MULTI-CHAMBERED AMPOULE FOR DISPENSING A MIXTURE COMPRISING SEVERAL SUBSTANCES
AMPOULE MULTICHAMBRE SERVANT A DISTRIBUER UN MELANGE CONSTITUE DE PLUSIEURS SUBSTANCES

(30) Priorität: 03.11.1999 DE 29919291 U
(43) Veröffentlichungstag der Anmeldung: 10.10.2001
(73) Patentinhaber: Dentaco Dentalindustrie und -marketing GmbH, 61352 Bad Homburg (DE)
(72) Erfinder: SOGARO, Alberto, 61476 Kronberg (DE)
(74) Vertreter: Lippert, Hans, Dipl.-Ing.
(86) Internationale Anmeldenummer: PCT/EP2000/010778
(87) Internationale Veröffentlichungsnummer: WO 2001/032242

(56) Entgegenhaltungen:
- WO-A-92/10425
- WO-A-99/17833
- FR-A- 2 070 358
- US-A- 2 168 686
- US-A- 3 572 336
- US-A- 4 303 069
- US-A- 4 610 666
- US-A- 5 599 312

## Beschreibung

Die Erfindung betrifft eine Mehrkammer-Ampulle zum Ausgeben eines aus mehreren Substanzen bestehenden Gemisches. In der Medizin und Kosmetik, aber auch auf anderen Gebieten, ist es bei einem Mehrkomponentenprodukt oft erforderlich, die einzelnen Komponenten separat voneinander aufzubewahren und erst unmittelbar vor der Anwendung miteinander zu vermischen. Darüber hinaus ist es oft erforderlich oder zumindest erwünscht, die einzelnen Komponenten möglichst dicht verschlossen aufzubewahren.

Aus der EP 0 090 413 A2 ist es bekannt, wenigstens zwei bei der Anwendung miteinander zu vermischende flüssige, fluide oder semifluide Substanzen in separaten Kartuschen aufzubewahren, die zu einer Einheit aus mehreren parallel zueinander angeordneten Kartuschen zusammengefaßt sind. Die Kartuschen sind aus einer weichen Kunststofffolie hergestellt und haben nur an ihrem vorderen Ende eine Öffnung, aus der die in der Kartusche aufbewahrte Substanz austreten kann. Das vordere Kartuschenende läuft im allgemeinen in einer konisch zulaufenden Spitze aus, deren Austrittsöffnung mit einer Kappe verschlossen ist. Zur Anwendung wird eine aus beispielsweise zwei Kartuschen bestehende Einheit in ein Gerät eingesetzt, das mit einem verschiebbaren Doppelkolben ausgerüstet ist, mit dem auf die beiden Kartuschen eingewirkt werden kann. Vor der Betätigung des Doppelkolbens werden die beiden Verschlußkappen abgenommen, und es wird eine gemeinsame Mischvorrichtung auf die rohrförmigen Austrittsabschnitte der beiden Kartuschen gesteckt. Die Mischvorrichtung hat zwei Eintrittskanäle_und einen gemeinsamen Austrittskanal, der im Inneren der Mischvorrichtung mit den beiden Eintrittskanälen in Verbindung steht. Die Gesamtanordnung aus den Kartuschen und den Gerätschaften zum Auspressen der Kartuschen und Mischen der ausgepreßten Substanzen ist relativ kompliziert aufgebaut und nicht einfach zu bedienen.

Eine vom Prinzip her sehr ähnliche Dosier- und Mischeinrichtung ist aus der EP 0 313 519 A1 bekannt. Hier werden zwei Einzelkartuschen in ein Gerät eingesetzt, das mit einer maschinellen Auspzeßeinheit aus zwei miteinander verbundenen Kolbenstangen ausgerüstet ist.

Im Gerätegehäuse ist eine Halterung für die einzusetzenden Kartuschen vorgesehen. An diese Halterung schließt sich eine Mischvorrichtung mit einer Mischkammer an, in die sich ein mit den Kartuschen austauschbares Mischorgan erstreckt. Auch hier ist die Mischkammer über zwei Eintrittskanäle in Form von zwei Verbindungsleitungen mit den Kartuschen verbunden. Zu diesem Zweck haben die Kartuschen jeweils ein verjüngtes Auslaßende, das beim Einsetzen der Kartuschen in das Gerät an die Verbindungsleitungen fest anzuschließen ist. Die Kartuschen sind am Auslassende mit einem membranartigen Verschluß versehen, welcher bei Inbetriebnahme der Auspreßeinheit unter Druck bricht. Alternativ ist am kartuschenseitigen Ende der Verbindungsleitungen jeweils ein rohrförmiges Kupplungsteil mit einer abgeschrägten ringförmigen Schneide vorgesehen, die beim Einsetzen der Kartuschen in das Gerät die am Kartuschenauslaßende vorgesehene Membran durchschneidet. Am entgegengesetzten Ende sind die Kartuschen mit einem Aufreißdeckel verschlossen, der vor dem Einsetzen der Kartuschen in das Gerät entfernt wird. Danach ist das der Auspreßeinheit zugewandte Kartuschenende nur noch durch einen in die Kartusche verschiebbar eingesetzten, kolbenartigen Einsatz abgedichtet. Diese Dosier- und Mischeinrichtung ist ebenfalls kompliziert aufgebaut und nicht einfach zu bedienen.

Ferner sind eine Vielzahl Spritzengamituren vorgeschlagen worden, die die Ausgabe gemischter Substanzen aus in die Spritzengarnitur eingesetzten Ampullen ermöglichen. In der Regel weisen hier die Kartuschen auf ihrer Austrittsseite Membrane auf, die im Betrieb von Hohlnadeln durchstochen werden, welche über entsprechende Verbindungskanäle in eine gemeinsame hohle Austrittsnadel münden. Die Verbindungskanäle sind teilweise noch mit Ventilen versehen, um einen sicheren dichten Übertritt der Reagentien in die gemeinsame Austrittsnadel zu ermöglichen. Dazu wird auf die WO 92/10425, US 5 314 412, US 5 599 312 verwiesen. In der US 5 542 934 münden die hohlen Durchstechnadeln in eine Kammer, in der sich die Substanzen vermischen und die durch eine auf das Kartuschengehäuse aufgeschraubte Kappe gebildet ist. In der AT 366 916 sind zwei Spritzenkörper in eine gemeinsame Halterung eingesetzt. Die Konusse der Spritzenkörper ragen in Einsteck-Konusse eines Sammelkopfes hinein. Innerhalb des Sammelkopfes führt von jedem Konus ein eigener Förderkanal bis in einen am Sammelkopf vorgesehenen Konuskopf mit Hohlnadel. In der AT 400 675 B ist eine Spritzengarnitur beschrieben, in der die Spritzenkörper mit einem Verbindungssteg zu einer unzerlegbaren plattenförmigen Spritzeneinheit verbunden sind, in die eine Kolbeneinheit eingesetzt wird. Ferner ist ein auf die Spritzeneinheit aufsteckbares Ausgabeteil vorgesehen, das nach Abnahme eines kappenartigen Verschlusses von der Spritzeneinheit auf diese aufgesteckt wird. Die Spritzeneinheit ist rückseitig durch bewegliche Kolbenstopfen verschlossen. Diese weisen eine Schnapp- oder Rastverbindung für die vorderen Enden von Kolbenstangen auf. Die Konusse der Spritzenkörper münden nach Aufstecken der Ausgabeeinheit in entsprechende konusförmige Ausnehmungen, die in separate Kanäle übergehen. Die Kanäle münden in eine herkömmliche Mischkanüle, die auf das Abgabeteil aufgesteckt ist.

Im ersten Teil des Anspruchs 1 ist ein Stand der Technik nach der bereits oben erwähnten US 5 599 312 berücksichtigt. Aus diesem Dokument ist eine Spritze mit einem Doppelkammerbehälter bekannt. Am vorderen Ende des Behälters sind zwei im wesentlichen zylindrische Stutzen ausgebildet, durch die sich Austrittsöffnungen vom Inneren der Kammern nach aussen erstrecken. Vor Ingebrauchnahme der Spritze wird auf die beiden Stutzen ein Hohlnadelkörper gesteckt, in dem zwei Eintrittsleitungen und eine Austrittsleitung ausgebildet sind. Die Eintrittsleitungen haben an ihren Eintrittsstellen zu den Stutzen komplementäre Ausnehmungen, die zum festen Aufstecken des Hohlnadelkörpers auf die Stutzen dienen. Die beiden Eintrittsleitungen sind im Inneren des Hohlnadelkörpers zusammengeführt und gehen hinter ihrer Zusamrnenführung in die mit einer Hohlnadel verbundene Austrittsleitung über. Der mit den beiden Eintrittsleitungen und der Austrittleitung durchsetzte Hohlnadelkörper hat einen komplizierten Aufbau und verursacht relativ hohe Herstellungskosten.

Aufgabe der Erfindung ist es, eine möglichst einfach ausgebildete und leicht zu handhabende Mehrkammer-Ampulle zum Ausgeben eines aus mehreren Substanzen bestehenden Gemisches zu schaffen.

Diese Aufgabe wird durch den Gegenstand des Anspruchs 1 gelöst. Bevorzugte Weiterbildungen der Erfindung sind in den Unteransprüchen definiert. Die danach geschaffene Mehrkammer-Ampulle hat den Vorteil, daß sie keine separaten Geräte zum Ausbringen und Mischen der Substanzen benötigt. Vielmehr sind die Ausbring- und Mischmittel in die eigentliche Mehrkammer-Ampulle integriert und erfüllen darin weitere Funktionen. So dienen in den Kammern verbleibende Kolben zum Abschließen der hinteren Öffnungen der die Substanzen aufbewahrenden Kammern, wobei diese Kolben in einer bevorzugten Ausführung die vorderen Enden einer einstückigen Kolbeneinheit sind und nicht zusätzlich vorgesehene, gesonderte Kolbenstopfen, zu deren Verschiebung eine separate Kolbenstangeneinheit erforderlich ist. Die Mischvorrichtung bildet gleichzeitig das Gehäuse zur Aufnahme des mit den Kammern versehenen Behälters, und dieses Gehäuse dichtet zusammen mit dem Behälter den Mischraum hinsichtlich unerwünschter Leckstellen ab. Somit sind zu entfernende Verschlußkappen und anschließend aufzusteckende Ausgabeeinheiten nicht erforderlich. Hierdurch ist einerseits das Bedienerfordernis des Ausrichtens, Aufsteckens und Einpassens einer Ausgabeeinheit vermieden. Andererseits entfällt auch die Gefahr undichter Stellen, die entstehen können, wenn die anzubringenden Einheiten nicht richtig verbunden werden.

In der erfindungsgemäßen Ampulle kann der Behälter mit den Kammern ohne zusätzliche Dichtungselemente in den Innenraum allein durch enges Anliegen der Wandungen von Behälter und Gehäuse über einen längeren Wandabschnitt gewährleistet werden. Daher kann der Austritt der zu mischenden Substanzen direkt aus den Kammern des Behälters in den Mischraum erfolgen. Zusätzliche Leitungen oder Kanäle bzw. dicht einzusetzende Konusstücke sind vermieden.

Das einzige Bedienerfordemis für die Inbetriebnahme der Ampulle besteht in der Erfindung gegebenenfalls darin, vor Inbetriebnahme ein Verschlußmittel an den vorderen Austrittsöffnungen der Kammern zu öffnen. Bei einer Ausführung wird hierzu eine seitliche aus dem Gehäuse herausgeführte Verschlußfolie einfach abgezogen. Dies kann mit einer Hand geschehen. Bei einer anderen Ausführung wird die Verschlußfolie durch Gehäusevorsprünge automatisch durchstochen, wenn der Behälter beim Vorschieben der Kolben in Richtung der Mischkammer nach vorne geschoben wird. Im Gegensatz zu den aus dem Stand der Technik bekannten hohlen Durchstechnadeln oder rohrförmigen Kupplungsstücken mit Schneide bilden diese Gehäusevorsprünge in der Verschlußfolie keine rundum gegenüber der Folie dichten Durchtrittskanäle, sondern dienen lediglich zur Perforation der Folie.

Bei einer weiteren Ausführung ist das Verschlußmittel eine Art Stöpsel, der derart ausgebildet ist, daß er zum Aktivieren der Ampulle beim Vorschieben des Kammerbehälters in Richtung auf die Mischkammer mit dem Gehäuse in Anschlag gerät und dadurch unter Freigabe der Kammeraustrittsöffnung in das Innere der Kammer gedrückt wird. Hierbei kann der Kammerbehälter von Hand unmittelbar oder mittelbar durch Druckausübung auf die Kolbeneinheit gegen das Gehäuse gedrückt werden. Vorzugsweise sind für jede Kammer keine gesonderten Stöpsel, sondern eine gemeinsame Stöpseleinheit vorgesehen, beispielsweise ein Doppelstöpsel bei einem Behälter mit zwei Substanzkammem.

Bevorzugte Ausführungsbeispiele der Erfindung werden nachstehend anhand von Zeichnungen erläutert. Es zeigen:
FIG. 1 eine teilweise geschnittene, im Aufriß dargestellte Zusammenbauansicht eines ersten Ausführungsbeispiels der Erfindung,
FIG. 2 eine teilweise geschnittene Ansicht des ersten Ausführungsbeispiels von FIG. 1 in einem zusammengefügten Zustand, in welchem die Substanzen in den Kammern abgedichtet aufbewahrt werden,
FIG. 3 eine geschnittene Ansicht des ersten Ausführungsbeispiels im zusammengefügten Zustand längs der Schnittlinie 3-3 in FIG.2,
FIG. 4 eine Ansicht auf ein bei dem dargestellten Ausführungsbeispiel verwendetes Verschlußmittel,
FIG. 5 eine teilweise geschnittene, im Aufriß dargestellte Zusammenbauansicht eines zweiten Ausführungsbeispiels der Erfindung,
FIG. 6 eine teilweise geschnittene Ansicht des beim zweiten Ausführungsbeispiel nach FIG. 5 benutzten Substanzenbehälters im verschlossenen Zustand,
FIG. 7 eine geschnittene Ansicht eines beim zweiten Ausführungsbeispiel benutzten Verschlußmittels längs der Schnittlinie 7-7 in FIG. 6,
FIG. 8 eine teilweise geschnittene, weggebrochene Ansicht des zweiten Ausführungsbeispiels im aktivierten Zustand, und
FIG. 9 eine geschnittene, weggebrochene Ansicht wesentlicher Teile eines dritten Ausführungsbeispiels der Erfindung.

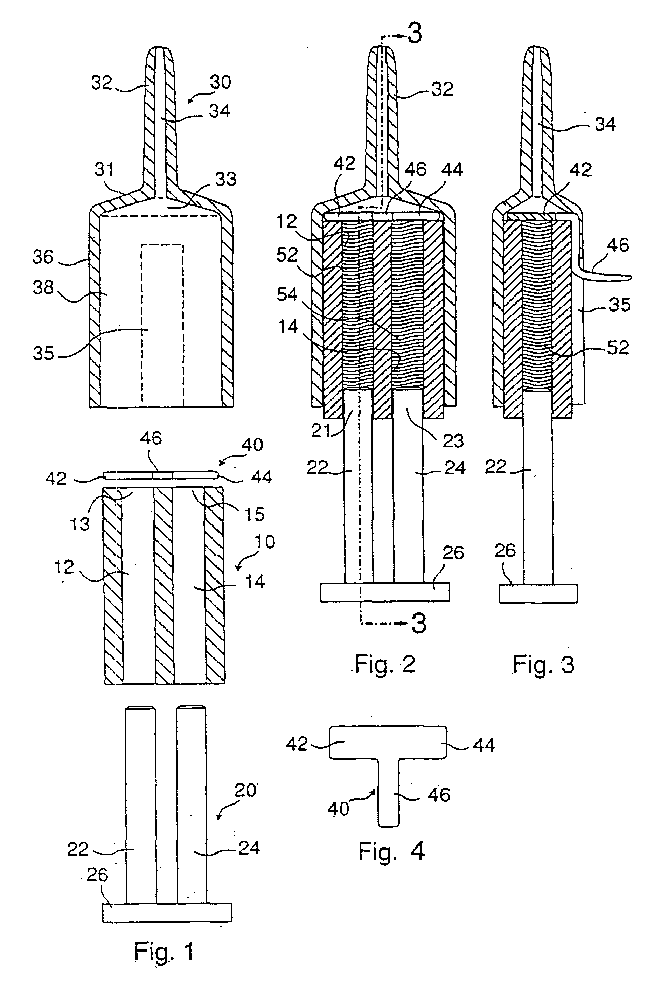

Das in den Figuren 1 bis 4 dargestellte erste Ausführungsbeispiel einer Mehrkammer-Ampulle nach der Erfindung besteht im wesentlichen aus drei Teilen, nämlich einem Behälter 10, einer Kolbenanordnung 20 und einem Gehäuse 30. Im Behälter 10 sind zwei separate zylindrische Kammern 12 und 14 ausgebildet, die sich in Axialrichtung des Behälters 10 erstrekken und sowohl an ihrem hinteren (unteren) Ende als auch an ihrem vorderen (oberen) Ende offen sind.

Die Kolbenanordnung 20 besteht aus zwei in einem Abstand parallel zueinander angeordneten kreiszylindrischen Kolben 22 und 24, deren hintere Enden durch eine Platte 26 zu einer Kolbeneinheit miteinander verbunden sind. Die Kolben 22 und 24 sind koaxial mit den Kammern 12 und 14 ausgerichtet, und der Außendurchmesser der Kolben 22 und 24 entspricht im wesentlichen dem Innendurchmesser des kreiszylindrischen Querschnitts der Kammern 12 und 14, so daß sich die in die Kammern 12 und 14 eingesetzten Kolben 22 und 24 möglichst dicht, insbesondere flüssigkeitsdicht, an die Kammerwandungen anlegen, jedoch noch in axialer Richtung verschieben lassen. Um die beiden sich widersprechenden Funktionen zu erfüllen, können die vorderen Enden 21 und 23 der Kolben 22 und 24 im Vergleich zur Restlänge der Kolben geringfügig überdimensioniert sein.

Das Gehäuse 30 besteht aus einem vorderen Abschnitt 32 und einem hinteren Abschnitt 36. Der vordere Abschnitt 32 ist rohrförmig ausgebildet und umschließt einen Austrittskanal 34 mit einem relativ kleinen kreisrunden Querschnitt. Der hintere Abschnitt 36 des Gehäuses 30 hat wesentlich größere Querabmessungen als der vordere Abschnitt 32 und begrenzt einen Innenraum 38, der nach hinten offen ist und in Richtung nach vome in den Austrittskanal 34 übergeht. Der Innenraum 38 ist mit dem Behälter 10 koaxial ausgerichtet. Der Behälter 10 hat quer zur Axialrichtung eine äußere Querschnittsfläche, die im wesentlichen mit der inneren Querschnittsfläche des Hohlraums 38 übereinstimmt, so daß sich der in den Innenraum 38 von hinten her eingeschobene Behälter 10 mit seiner Außenwand möglichst dicht, insbesondere flüssigkeitsdicht, an die den Innenraum 38 begrenzende Umfangswand des Gehäuses 30 anlegt. Zwischen dem rohrförmigen vorderen Abschnitt 32 kleinen Durchmessers und dem dagegen aufgeweiteten hinteren Abschnitt 36 mit größeren Querschnittsabmessungen weist das Gehäuse 30 eine im wesentlichen radial verlaufende Schulter 31 auf, die einen sehr flachen kegelstumpfförmigen Raum 33 begrenzt, der einen Übergangsraum zwischen dem weiten Innenraum 38 und dem relativ engen Austrittskanal 34 darstellt und auch Austrittsoder Mischraum genannt wird. Die Schulter 31 ist gemäß FIG. 1 und 2 abgeschrägt und bietet eine Angriffsfläche für die Ausübung eines Fingerdrucks, wobei mit einem anderen Finger derselben Hand auf die Kolbenanordnung gedrückt werden kann, so daß zur Entnahme des Gemisches die Kolbenanordnung und das Gehäuse teleskopartig zusammenschiebbar sind.

In FIG. 1 ist noch ein Verschlußmittel 40 eingezeichnet, das in FIG. 4 in einer Ansicht von oben dargestellt ist und zwei Verschlußabschnitte 42 und 44 sowie einen Abziehabschnitt 46 aufweist.

FIG. 2 und 3 zeigen das Ausführungsbeispiel der erfindungsgemäßen Mehrkammer-Ampulle in einem Zustand, in welchem die Ampulle versandt oder gelagert werden kann, im übrigen aber sofort einsatzfähig ist. In dem in FIG. 2 und 3 dargestellten Lager- und Transportzustand ist das vordere Ende der Kammern 12 und 14 mit dem Verschlußelement 40 abgeschlossen bzw. abgedichtet. Bei dem Verschlußelement 40 kann es sich beispielsweise um eine Folie handeln, die auf die vordere Stirnfläche des Behälters 10 geklebt ist und dabei bevorzugt als Kunststoff-Folie oder mit einer entsprechenden Kunststoffbeschichtung mit der Stirnfläche verschweißt ist. Hierzu können aus der Kunststofftechnik hinreichend bekannte Heißversiegelungsverfahren und dergleichen angewandt werden. Das hintere Ende der Kammern 12 und 14 ist durch die Kolben 22 und 24 abgeschlossen oder abgedichtet, deren vordere Enden 21 und 23 ein gewisses, im Vergleich zur Länge der Kammern 12, 14 kurzes Stück in die Kammern 12 und 14 eingeschoben sind. Das Verschlußmittel 40 und die Kolben 22 und 24 begrenzen in den Kammern 12 und 14 jeweils einen fluid- oder flüssigkeitsdichten Raum. In diesen Räumen befinden sich die beim Ausgeben miteinander zu vermischenden Substanzen 52 und 54.

Bei dem betrachteten ersten Ausführungsbeispiel ist, wie es aus FIG. 3 hervorgeht und in FIG. 1 durch gestrichelte Linien angedeutet ist, in einer Seitenwand des hinteren Abschnitts 36 des Gehäuses 30 ein schlitzförmiger Ausschnitt 35 vorgesehen, durch den der Abziehabschnitt 46 des Verschlußmittels 40 nach außen geführt ist. Diese Maßnahme ermöglicht es, daß zur gemeinsamen Anwendung der Substanzen bzw. zur Ingebrauchnahme der Mehrkammer-Ampulle das Verschlußmittel 40 in dem in FIG. 2 und 3 dargestellten zusammengefügten Zustand der Ampulle abgezogen werden kann, wodurch die oberen Öffnungen 13 und 15 der Kammern 12 und 14 freigelegt werden. In diesem Zusammenhang ist zu beachten, daß die als Verschlußmittel 40 eingesetzte Folie derart dünn ist, daß bei und nach dem Abziehen der Folie durch den Ausschnitt 35 keine beachtliche Leckstelle zwischen der Außenwand des Behälters 10 und der den Innenraum 38 umgebenden Wand des hinteren Abschnitts 36 des Gehäuses 30 auftritt. Zudem wird durch den zur Ingebrauchnahme erforderlichen Druck auf die Kolbenanordnung 20 der vordere Abschnitt des Behälters 10 fest gegen die Innenwand des Gehäuses 30 gedrückt, die sich im Bereich der Schulter 31 nach vorne verjüngt. Das vordere Ende des Behälters 10 schließt daher den Raum 33 gegenüber seitlichen Leckstellen sicher ab, so daß die Substanzen 52 und 54, die unter der Einwirkung der Vorschubkraft der Kolbenanordnung 20 aus den vorderen Öffnungen der Kammern 12 und 14 austreten, über den Raum 33 in den Austrittskanal 34 gelangen und an dessen vorderen Ende austreten. Hierbei werden im Raum 33 und im Austrittskanal 34 die beiden Substanzen 52 und 54 miteinander vermischt. Die Mischwirkung kann noch dadurch gesteigert werden, daß im Austrittskanal 34 ein statisches Mischelement (nicht gezeigt) vorgesehen ist. Derartige Mischelemente sind allgemein bekannt und können beispielsweise die Form einer Spirale haben.

Das Verschlußmittel 40 könnte man auch in einer Ausführung ohne den Abziehabschnitt 46 anwenden. Die Abschnitte 42 und 44 wären dann so ausgestaltet und ausgelegt, daß sie bei Ausübung von Druck auf die Kolbenanordnung 20 weggedrückt werden und die vorderen Öffnungen 13 und 15 der Kammern 12 und 14 freigeben. Das Verschlußmittel 40 kann auch in Form einer dünnen Haut oder Membran vorliegen, die in den Behälter 10 integriert ist und bei Ausübung von Druck aufplatzt. Des weiteren können an der Innenwand der Schulter 31 nach hinten ragende spitze Vorsprünge ausgebildet sein, die bei Ausübung von Druck auf die Kolbenanordnung 20 in Richtung nach vorne die Haut oder Membran durchstechen, die die oberen Öffnungen 13 und 15 der Kammern 12 und 14 überspannt. Für diese Ausführungsform ist es von Vorteil, wenn der Behälter 10 im Gehäuse 30 so verschiebbar ist, daß er sich bei Ausübung von Druck auf die Kolbenanordnung nach vorne bewegt und dadurch die Haut oder Membran in die spitzen Vorsprünge hineingeschoben wird. Alternativ kann das Öffnen oder Durchstechen der Haut auch durch direkte Druckausübung auf den Behälter 10 bewerkstelligt werden. Bei der Anwendung von solchen oder anderen Verschlußmitteln ohne einen Abziehabschnitt 46 entfällt der Ausschnitt 35.

Die Teile 10, 20 und 30 der Mehrkammer-Ampulle sind vorzugsweise aus einem thermoplastischen Kunststoff hergestellt, beispielsweise Polyethylen. Hierbei kann die Kolbenanordnung 20 aus einem härteren Kunststoff bestehen, wohingegen der Behälter 10 und auch das Gehäuse 30 eine gewisse Elastizität haben sollten.

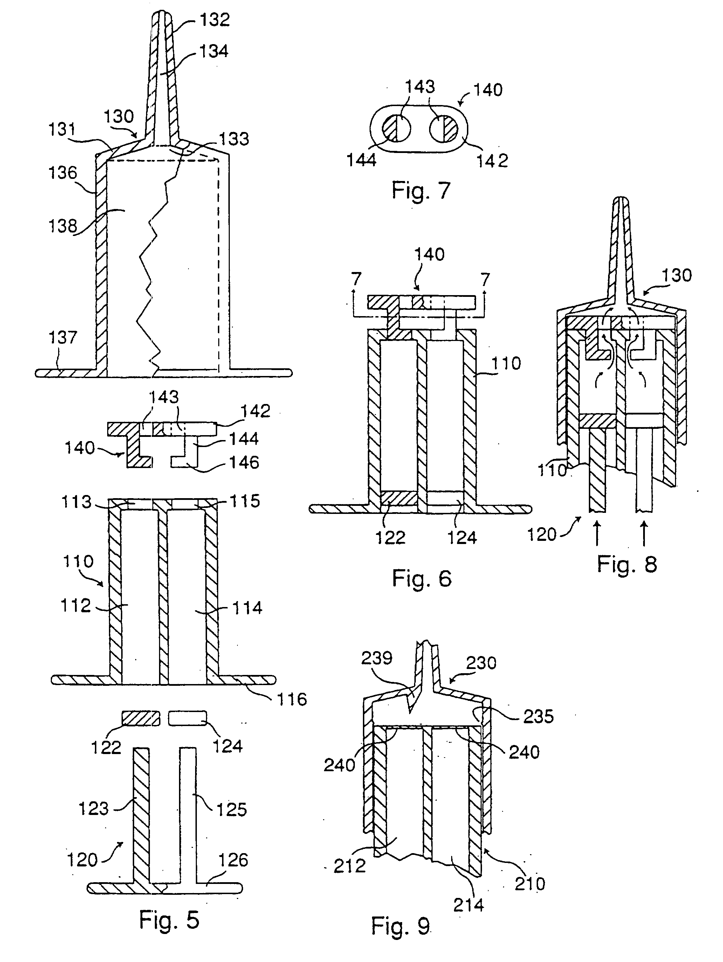

Das in den Figuren 5 bis 8 dargestellte zweite Ausrührungsbeispiel einer Mehrkammer-Ampulle nach der Erfindung besteht, wie das erste Ausführungsbeispiel, im wesentlichen aus drei Teilen, nämlich einem Behälter 110, einer Kolbenanordnung 120 und einem Gehäuse 130. Im Behälter 110 sind zwei separate zylindrische Kammern 112 und 114 ausgebildet, die sich in Axialrichtung des Behälters 110 erstrecken und sowohl an ihrem hinterem als auch an ihrem vorderen Ende offen sind. Am unteren Ende des Behälters 110 ist ein radial nach außen weisender Ansatz 116 angeformt.

Die Kolbenanordnung 120 besteht aus zwei separaten, kreiszylindrischen Kolben 122 und 124 sowie aus zwei in einem Abstand parallel zueinander angeordneten Kolbenstangen 123 und 125, deren hintere Enden durch eine Platte 126 miteinander verbunden sind. Der Außendurchmesser der Kolben 122 und 124 entspricht im wesentlichen dem Innendurchmesser des kreiszylindrischen Querschnitts der Kammern 112 und 114, so daß sich die in die Kammern 112 und 114 eingesetzten Kolben 122 und 124 möglichst dicht, insbesondere flüssigkeitsdicht, an die Kammerwandungen anlegen, jedoch noch in axialer Richtung verschieben lassen. Zu diesem Zweck können die Kolben 122 und 124 im Vergleich zum Querschnitt der Kammern 112 und 114 geringfügig überdimensioniert sein.

Das Gehäuse 130 des zweiten Ausführungsbeispiels ist im wesentlichen wie das Gehäuse 30 des ersten Ausführungsbeispiels ausgebildet. Einander entsprechende Gehäuseteile sind deshalb in FIG. 4 mit Bezugszahlen versehen, denen im Vergleich zu FIG. 1 jeweils eine "1" vorangestellt ist. Da beim zweiten Ausführungsbeispiel ein anderes Verschlußmittel 140 vorgesehen ist, entfällt beim Gehäuse 130 der beim Gehäuse 30 gegebenenfalls vorhandene Ausschnitt 35. Zur besseren Handhabung ist zusätzlich am hinteren Ende des Gehäuses 130 ein radial nach außen weisender Ansatz 137 angeformt.

Das zum dichten Verschließen der oberen Öffnungen 113 und 115 der Kammern 112 und 114 dienende Verschlußmittel 140 ist als Stöpsel ausgebildet. Das Verschlußmittel besteht aus einer Platte 142, deren äußere Querabmessungen mit denjenigen des Behälters 110 bzw. mit den Querabmessungen des Innenraums 138 im wesentlichen übereinstimmen. In der Platte 142 sind zwei halbkreisförmige Durchgangsöffnungen 143 vorgesehen. Angrenzend an die Durchgangsöffnungen 143 ragen von der Unterseite der Platte zwei Beine 144 nach unten. die einen halbkreisförmigen Querschnitt haben und jeweils an ihrem unteren Ende in einen zylindrischen Stopfen 146 übergehen. Die Stopfen 146 dienen zum flüssigkeitsdichten Verschließen der oberen Öffnungen 113 und 115 und haben deshalb in Bezug auf den Innendurchmesser der Öffnungen 113 und 115 einen entsprechend bemessenen Außendurchmesser.

FIG. 6 zeigt den Behälter 110 im gebrauchsfertigen Zustand. In diesem Zustand sind die oberen Öffnungen 113 und 115 der Kammern 112 und 114 durch die Stopfen 146 des Verschlußmittels 140 flüssigkeitsdicht verschlossen. Das untere Ende der Kammern 112 und 114 ist durch die darin eingesetzten Kolben 122 und 124 abgeschlossen. Das Verschlußmittel 140 und die Kolben 122 und 124 begrenzen somit in den Kammern 112 und 114 jeweils einen fluid- oder flüssigkeitsdichten Raum. In diesen Räumen befinden sich die beim Ausgeben miteinander zu vermischenden Substanzen (nicht gezeigt).

Der in FIG. 6 dargestellte gebrauchsfertige Behälter 110 kann separat vom Gehäuse 130 gelagert und transportiert werden. Vorzugsweise wird jedoch auch das in FIG. 5 bis 8 dargestellte zweite Ausführungsbeispiel der Erfindung in einem solchen Zustand gelagert und transportiert, in welchern der gebrauchsfertige Behälter 110 bereits in den Innenraum 138 des Gehäuses 130 eingeschoben ist, allerdings nur soweit, daß das Verschlußmittel 140 von der Schulter 131 noch beabstandet ist oder nur ganz leicht an der Schulter 131 anliegt. Die Mehrkammer-Ampulle ist deshalb noch nicht aktiviert.

Unmittelbar vor Ingebrauchnahme der Mehrkammer-Ampulle erfolgt die Aktivierung dadurch, daß durch vollständiges Einschieben des Behälters 110 in den Innenraum 138 des Gehäuses 130 das Verschlußmittel 140 gegen die Schulter 131 gedrückt wird, wodurch die Stopfen 146 aus den Öffnungen 113 und 115 nach innen in die Kammern 112 und 114 gelangen und die Platte 142 in Anlage mit der oberen Stirnfläche des Behälters 110 kommt, wie es in FIG. 8 dargestellt ist. Da die Kammern 112 und 114 eine größere lichte Weite als die Öffnungen 113 und 115 haben bzw. der Außendurchmesser der Stopfen 146 geringer als der Innendurchmesser der Kammern 112 und 114 unterhalb der Öffnungen 113 und 115 ist, sind die oberen Enden der Kammern 112 und 114 nicht mehr verschlossen und die darin befindlichen Substanzen können durch die Öffnungen 113 und 115 sowie die sich jetzt unmittelbar daran anschließenden Durchgangsöffnungen 143 in den Austritts- oder Mischraum 131 gelangen. Zum Erleichtern der Aktivierung mit den Fingern dient der am hinteren Ende des Behälters 110 ausgebildete Ansatz 116, wobei andere Finger der selben Hand am Ansatz 137 oder an der Schulter 131 des Gehäuses 130 angreifen können. Für einen Fachmann ist es augenscheinlich, daß die Ansätze 116 und 137 zum Vornehmen der gewünschten Funktion in manigfacher Weise ausgebildet sein können, beispielsweise auch in Form eines nur an einer Stelle der jeweiligen Umfangswand vorgesehenen Vorsprungs oder in Form zwei einander gegenüberliegender Vorsprünge.

Zum Ausgeben der Substanzen werden dann im aktivierten Zustand die Kolben 122 und 124 mittels der über die Platte 126 miteinander verbundenen Kolbenstangen 123 und 125 in Richtung nach vorne in die Kammern 112 und 114 geschoben. Dabei werden die Substanzen in den Austrittstaum 133 gedrückt und von dort über den Austrittskanal 134 ausgegeben, wie es in FIG. S durch eingezeichnete Pfeile angedeutet ist.

Im Austrittskanal 134 kann wahlweise, wie beim ersten Ausführungsbeispiel, ein statischer Mischer (nicht gezeigt) angeordnet sein. Gleichermaßen kann man, wie beim ersten Ausfiihrungsbeispiel, anstelle separater Kolben 122 und 124 sowie zugehöriger Kolbenstangen 123 und 124 eine einstückige Kolbenanordnung wie beim ersten Ausführungsbeispiel verwenden. Gleichermaßen kann man auch beim ersten Ausführungsbeispiel separate Kolben mit zugehörigen Kolbenstangen verwenden.

Das dargestellte Verschlußmittel 140 hat den Vorteil, daß nach der Aktivierung in dem in FIG. 8 dargestellten Zustand die zwischen der oberen Stirnseite des Behälters 110 und der Unterseite der Schulter 131 eingeklemmte Platte 142 dafür Sorge trägt, daß die Stopfen 146 bei der Vorschubbewegung der Kolben 122 und 124 die Öffnungen 113 und 115 nicht mehr verschließen können, da sie von den Beinen 144 in einem Abstand von den Öffnungen 113 und 115 gehalten werden. Gleichzeitig kann die Platte 142 die Abdichtung zwischen der Außenwand des Behälters 110 und der Innenwand des Gehäuses 130 fordern. Zu diesem Zweck kann das Verschlußmittel 140 aus einem gummiartigen Werkstoff hergestellt sein.

Für einen Fachmann ist es augenscheinlich, daß das Verschlußmittel 140 in mannigfacher Weise abgewandelt werden kann. Dies gilt insbesondere für die Geometrie der Platte 142, der Durchgangsöffnungen 143 und der Beine 144.

Bezüglich der Materialien für die einzelnen Teile des zweiten Ausführungsbeispiels kann auf die entsprechenden Angaben zum ersten Ausführungsbeispiel verwiesen werden.

Wesentliche Teile eines dritten Ausführungsbeispiels sind in FIG. 9 dargestellt. Die Kammern 212 und 214 des Behälters 210 sind dort an ihrem oberen Ende mit einer Haut oder Membran 240 verschlossen. Im Inneren des Gehäuses 230 ist ein spitzer Vorsprung 239 ausgebildet. Zum Aktivieren wird der Behälter 210 über eine radial nach innen springende Lippe 235 nach oben in Richtung auf den spitzen Vorsprung 239 geschoben. Dabei durchsticht der Vorsprung 239 die Membran 240, so daß die in den Behälterkammern befindliche Substanz mit einer nicht dargestellten Kolbenanordnung ausgegeben werden kann. Die Lippe 235 fördert die Abdichtung im aktivierten Zustand und dient gleichzeitig dazu, bei der Vorschubbewegung der Kolbenanordnung eine Verschiebung des Behälters 210 nach hinten infolge des Druckaufbaus zu verhindern.

Es ist für einen Fachmann augenscheinlich, daß auch beim ersten und zweiten Ausführungsbeispiel mit der Lippe 235 vergleichbare Mittel vorgesehen sein können, um die Abdichtung des Austritts- oder Mischraumes 33 und 133 zu fördern und, insbesondere, eine Rückbewegung des Behälters 10 und 110 infolge des Druckaufbaus im Raum 33 und 133 zu vermeiden. Zu diesem Zweck genügt es im allgemeinen, wenn an der Innenwand des Gehäuses 130 eine radial nach innen springende umlaufende Wulst oder an der Außenwand des Behälters 210 eine radial nach außen springende umlaufende Wulst vorgesehen ist. Im Bedarfsfall kann auch eine Rastung vorgesehen sein.

## Patentansprüche

1. Mehrkammer-Ampulle zum Ausgeben eines aus mehreren Substanzen bestehenden Gemisches, enthaltend:
einen länglichen Behälter (10; 110; 210) mit wenigstens zwei parallel zueinander angeordneten, zylindrischen Kammern (12, 14; 112, 114; 212, 214), die sich in der Axialrichtung des Behälters (10; 110; 210) von einem hinteren bis zu einem vorderen Ende des Behälters erstrecken und von denen jede ein offenes hinteres Ende und ein mit einer Austrittsöffnung (13, 15; 113, 115) versehenes vorderes Ende hat,
jeweils einen in jede der Kammern (12, 14; 112, 114; 212, 214) in Axialrichtung verschiebbar einsetzbaren Kolben (22, 24; 122, 124), der im eingesetzten Zustand eine Abdichtung gegenüber der Kammerinnenwand vorsieht, und
ein Gehäuse (30; 130; 230) mit einer Angriffsfläche (31; 313, 137) zum manuellen teleskopartigen Zusammenschieben von Kolben und Gehäuse, mit einem einen Austrittskanal (34; 134) umgebenden vorderen Abschnitt (32; 132) und einem an den vorderen Abschnitt angrenzenden hinteren Abschnitt (36; 136) mit einem darin ausgebildeten Innenraum (38; 138), in den der Behälter (10; 110; 210) verschieblich einsetzbar ist,
wobei in einem zur Anwendung der Mehrkammer-Ampulle bereiten Zustand die offenen hinteren Enden der Kammern (12, 14; 112, 114; 212, 214) durch die Kolben (22, 24; 122, 124) verschlossen sind, die Austrittsöffnungen (13, 15; 113; 115) am vorderen Ende der Kammern (12, 14; 112, 114; 212, 214) durch ein Verschlußmittel (40; 140; 240) verschlossen sind und in den vom Verschlußmittel (40; 140; 240) und den Kolben (22, 24; 122, 124) abgeschlossenen Kammerbereichen jeweils eine Substanz (52, 54) enthalten ist, **dadurch gekennzeichnet,**
**daß** der Behälter (10, 110, 210) durch eine einzige, im hinteren Gehäuseabschnitt (36, 136) ausgebildete Öffnung mit einem einem Aussenumfang des Behälters entsprechenden Innenumfang in den Innenraum (38, 138) einschiebbar ist, und
**daß** das vordere Behälterende, wenn der Behälter bei Aktivierung der Mehrkammer-Ampulle vollständig in das Gehäuse hineingeschoben ist, zusammen mit dem Innenraum (38, 138) einen Mischraum (33; 133) begrenzt und den Mischraum gegenüber seitlichen Leckstellen dicht abschließt, welcher Mischraum in den Austrittskanal (34; 134) übergeht und die im vorderen Behälterende vorgesehenen Austrittsöffnungen (13, 15; 113, 115) überspannt.

2. Mehrkammer-Ampulle nach Anspruch 1, bei der zum Ausgeben des Gemisches bei in den Innenraum (38; 138) des hinteren Abschnitts (36; 136) des Gehäuses (30; 130; 230) eingesetztem Behälter (10; 110; 210), nach oder unter gleichzeitiger Freigabe der Austrittsöffnungen (13, 15; 113; 115) am vorderen Ende der Kammern (12, 14; 112, 114; 212, 214) durch das Verschlußmittel (40; 140; 240), die Kolben (22, 24; 122, 124) nach vorne in Richtung auf das vordere Ende des Behälters (10; 110; 210) gedrückt werden, so daß aus den Kammern (12, 14; 112, 114; 212, 214) die Substanzen austreten und über den gemeinsamen Austrittskanal (34; 134) die gemischten Substanzen nach außen abgegeben werden.

3. Mehrkammer-Ampulle nach Anspruch 1 oder 2, bei der zwischen dem vorderen Abschnitt (32; 132) und dem hinteren Abschnitt (36; 136) des Gehäuses (30; 130) eine im wesentlichen radial verlaufende, vorzugsweise abgeschrägte Schulter (31; 131) vorgesehen ist.

4. Mehrkammer-Ampulle nach einem der vorangegangenen Ansprüche, bei der in einer Seitenwand des hinteren Abschnitts (36) des Gehäuses (30) ein schlitzförmiger Ausschnitt (35) zum Abziehen des Verschlußmittels (40) vorgesehen ist.

5. Mehrkammer-Ampulle nach einem der vorangegangenen Ansprüche, bei der im Gehäuse (230) ein spitzer Vorsprung (239) zum Durchstechen des Verschlußmittels (240) vorgesehen ist.

6. Mehrkammer-Ampulle nach einem der vorangegangenen Ansprüche, bei der das Verschlußmittel (40) eine abziehbare Folie ist.

7. Mehrkammer-Ampulle nach einem der Ansprüche 1 bis 5, bei der das Verschlußmittel (240) eine perforierbare Haut ist.

8. Mehrkammer-Ampulle nach einem der Ansprüche 1 bis 5, bei der das Verschlußmittel (140) ein bei der Aktivierung der Ampulle mittels des Gehäuses in die Kammer (112, 114) drückbarer Stöpsel ist.

9. Mehrkammer-Ampulle nach einem der Ansprüche 1 bis 5, bei der das Verschlußmittel (140) wenigstens zwei Stopfen (146) zum Verschliessen der wenigstens beiden Austrittsöffnungen (113, 115) der Kammern (112, 115) und eine die Stopfen miteinander verbindende Platte (142) aufweist, wobei bei der Aktivierung der Ampulle die Platte mittels des Gehäuses in Richtung auf die Kammern gedrückt wird und hierbei die Stopfen aus den Austrittsöffnungen nach innen in die Kammern gelangen.

10. Mehrkammer-Ampulle nach einem der vorangegangenen Ansprüche, bei der die Kolben (20, 22) an ihrem hinteren Ende miteinander zu einer einstückigen Kolbenanordnung (20) verbunden sind.

11. Mehrkammer-Ampulle nach einem der vorangegangenen Ansprüche, bei der an die Innenwand des Gehäuses (230) oder die Außenwand des Behälters eine umlaufende Wulst (235) angeformt ist.

## Claims

1. A multi-chamber ampoule for dispensing a mixture comprising several substances, including:
an oblong container (10; 110; 210) having at least two cylindrical chambers (12, 14; 112, 114; 212, 214) arranged parallel to one another and extending in the axial direction of the container (10; 110; 210) from a rear end to a frontal end of the container, each of said chambers having an open rear end and an open front end being provided with an outlet opening (13, 15; 113, 115);
a respective piston (22, 24; 122, 124) which can be slidingly inserted into each chamber (12, 14; 112, 114) in an axial direction and, in the inserted state, creating a seal with respect to the inner wall of the chamber, and
a housing (30; 130; 230) having an engagement surface (31; 313 , 137) for manually sliding together piston and housing in telescope like manner, having a front section (32; 132) surrounding a discharge channel (34; 134) and a rear section (36; 136) adjacent to the front section having an inner chamber (38; 138) formed therein, into which inner chamber the container (10; 110; 210) can slidingly be inserted;
with, in a ready-to-use state of the multi-chamber ampoule, the openings at the rear end of the chambers (12, 14; 112, 114; 212, 214) being closed by pistons (22, 24; 122, 124), the openings (13, 15; 113, 115) at the front end of the chambers (12, 14; 112, 114; 212, 214) being sealed by a sealing means (40; 140; 240), and a substance (52, 54) being contained in each chamber region closed by the sealing means (40; 140; 240) and the pistons (22, 24; 122, 124),
**characterized in that**
the container (10, 110, 210) is insertable into said inner chamber (38, 138) through a single opening being formed in the rear housing section (36, 136) having an inner circumference corresponding to the outer circumference of the container, and
that said frontal end of the container, when said container is fully inserted into the housing upon activation of the multi-chamber ampoule, defines a mixing chamber (33; 133) together with the inner chamber (38, 138) and tightly seals the mixing chamber against sidewards leakage points, which mixing chamber leads into the discharge channel (34; 134) and extends over the outlet openings (13, 15; 113, 115) being provided in the frontal end of the container.

2. The multi-chamber ampoule according to claim 1, wherein, when the container (10; 110; 210) has been inserted into the inner chamber (38; 138) of the rear section (36; 136) of the housing (30; 130; 230), the pistons (22, 24; 122, 124) are urged forward in direction of the front end of the container (10; 110; 210) in order to dispense the mixture such that the substances discharge from the chambers (12, 14; 112, 114; 212, 214) and the mixed substances are dispensed to the exterior via the common discharge channel (32; 132), which urging is done subsequently to or simultaneously with a clearing of the openings (13, 15; 113, 115) at the front end of the chambers (12, 14; 112, 114; 212, 214) from the sealing means (40; 140; 240).

3. The multi-chamber ampoule according to claims 1 or 2, wherein, between the front section (32; 132) and the rear section (36; 136) of the housing (30; 130), a shoulder (31; 131) is provided which essentially extends in a radial direction and is preferably beveled.

4. The multi-chamber ampoule according to any preceding claim, wherein a cut out in form of a slot (35) is provided in a side wall of the rear section (36) of the housing (30) for removing the sealing means (40).

5. The multi-chamber ampoule according to any preceding claim, wherein a sharp protrusion (239) for piercing the sealing means (240) is provided in the housing (230).

6. The multi-chamber ampoule according to any preceding claim, wherein the sealing means (40) is a peelable foil.

7. The multi-chamber ampoule according to one of the claims 1 through 5, wherein the sealing means (240) is a skin which can be perforated.

8. The multi-chamber ampoule according to one of the claims 1 through 5, wherein the sealing means (140) is a plug that can be urged into the chamber (112, 114) by means of said housing upon activation of the ampoule.

9. The multi-chamber ampoule according to one of the claims 1 through 5, wherein said sealing means (140) has at least two plugs (146) for closing said at least two outlet openings (113, 115) of said chambers (112, 115) and has a plate (142) connecting said plugs to each another, said plate, upon activation of the ampoule, being urged in direction towards the chambers by means of the housing whereby the plugs move inwardly from the outlet openings into the chambers.

10. The multi-chamber ampoule according to any preceding claim, wherein the pistons (20, 22) are connected to one another at their rear end forming a one-piece piston arrangement (20).

11. The multi-chamber ampoule according to any preceding claim, wherein a circular bead (235) is formed to the interior wall of the housing (230) or to the exterior wall of the container.

## Revendications

1. Ampoule multichambre destinée à distribuer un mélange constitué de plusieurs substances, comportant :
un récipient longitudinal (10 ; 110 ; 210) comprenant au moins deux chambres cylindriques (12, 14 ; 112, 114 ; 212, 214) disposées parallèlement l'une à l'autre, qui s'étendent dans la direction axiale du récipient (10 ; 110 ; 210) depuis une extrémité arrière jusqu'à une extrémité avant du récipient et parmi lesquelles chacune présente une extrémité arrière ouverte et une extrémité avant pourvue d'une ouverture de sortie (13, 15 ; 113, 115),
respectivement un piston (22, 24 ; 122, 124) pouvant être inséré de manière à pouvoir se déplacer dans la direction axiale dans chacune des chambres (12, 14 ; 112, 114 ; 212, 214), lequel piston comporte un moyen d'étanchéité, qui lorsque le piston est dans un état inséré, est en regard de la paroi intérieure de la chambre, et
un logement (30 ; 130 ; 230) comprenant une surface d'attaque (31 ; 313, 137) destinée à rapprocher manuellement et de manière télescopique le piston et le logement, comprenant une section avant (32 ; 132) entourant un canal de sortie (34 ; 134) et une section arrière (36 ; 136) adjacente à la section avant, comprenant un espace intérieur (38 ; 138) conçu à l'intérieur de celle-ci, espace dans lequel peut être inséré le récipient (10 ; 110 ; 210) de manière mobile,
les extrémités arrières ouvertes des chambres (12, 14 ; 112, 114 ; 212, 214) étant fermées par les pistons (22, 24 ; 122, 124) dans un état prêt à utiliser l'ampoule multichambre, les ouvertures de sortie (13, 15 ; 113, 115) étant fermées à l'extrémité avant des chambres (12, 14 ; 112, 114 ; 212, 214) par un moyen de fermeture (40 ; 140 ; 240) et respectivement une substance (52, 54) étant contenue dans les zones de chambre fermées par le moyen de fermeture (40 ; 140 ; 240) et les pistons (22, 24 ; 122, 124), **caractérisée en ce que**
le récipient (10, 110, 210) peut être introduit dans l'espace intérieur (38, 138) à travers une seule ouverture, conçue dans la section de logement arrière (36, 136), comprenant une périphérie intérieure correspondant à une périphérie extérieure du récipient et
**en ce que** l'extrémité avant du récipient, lorsque le récipient est complètement inséré dans le logement à l'occasion de l'activation de l'ampoule multi-chambre, délimite conjointement avec l'espace intérieur (38, 138) un espace de mélange (33 ; 133) et isole de manière étanche l'espace de mélange par rapport aux points de fuite latéraux, lequel espace de mélange débouche dans le canal de sortie (34 ; 134) et recouvre les ouvertures de sortie (13, 15 ; 113, 115) prévues dans l'extrémité avant du récipient.

2. Ampoule multi-chambre selon la revendication 1, dans laquelle pour distribuer le mélange, lorsque le récipient (10 ; 110 ; 210) est inséré dans l'espace intérieur (38 ; 138) de la section arrière (36 ; 136) du logement (30 ; 130 ; 230), après ou pendant la libération simultanée des ouvertures de sortie (13, 15 ; 113, 115) à l'extrémité avant des chambres (12, 14 ; 112, 114 ; 212, 214) par le moyen de fermeture (40 ; 140 ; 240), les pistons (22, 24 ; 122, 124) sont pressés vers l'avant en direction de l'extrémité avant du récipient (10 ; 110 ; 210), de sorte que les substances sortent des chambres (12, 14 ; 112, 114 ; 212, 214) et que les substances mélangées sont distribuées vers l'extérieur par l'intermédiaire du canal de sortie (34 ; 134) commun.

3. Ampoule multi-chambre selon la revendication 1 ou 2, dans laquelle un épaulement (31 ; 131) de préférence conique, s'étendant sensiblement dans le sens radial, est prévu entre la section avant (32 ; 132) et la section arrière (36 ; 136).

4. Ampoule multi-chambre selon l'une quelconque des revendications précédentes, dans laquelle une rainure (35) en forme de fente est prévue dans une paroi latérale de la section arrière (36) du logement (30) pour retirer le moyen de fermeture (40).

5. Ampoule multi-chambre selon l'une quelconque des revendications précédentes, dans laquelle une saillie pointue (239) est prévue dans le logement (230) pour perforer le moyen de fermeture (240).

6. Ampoule multi-chambre selon l'une quelconque des revendications précédentes, dans laquelle le moyen de fermeture (40) est un film retirable.

7. Ampoule multi-chambre selon l'une des revendications 1 à 5, dans laquelle le moyen de fermeture (240) est une pellicule perforable.

8. Ampoule multi-chambre selon l'une des revendications 1 à 5, dans laquelle le moyen de fermeture (140) est un bouchon pouvant être pressé par le logement dans la chambre (112, 114) lors de l'actionnement de l'ampoule.

9. Ampoule multi-chambre selon l'une des revendications 1 à 5, dans laquelle le moyen de fermeture (140) comprend au moins deux bouchons (146) destinés à fermer au moins les deux ouvertures de sortie (113, 115) des chambres (112, 115) et une plaque (142) reliant les bouchons, la plaque étant pressée par le logement en direction des chambres à l'occasion de l'actionnement de l'ampoule et dans ce cas, les bouchons parvenant depuis les ouvertures de sortie vers l'intérieur des chambres.

10. Ampoule multi-chambre selon l'une quelconque des revendications précédentes, dans laquelle les pistons (20, 22) sont reliés les uns aux autres au niveau de leur extrémité arrière pour former un agencement de piston (20) en une seule pièce.

11. Ampoule multi-chambre selon l'une quelconque des revendications précédentes, dans laquelle un renflement annulaire (235) est formé sur la paroi intérieure du logement (230) ou la paroi extérieure du récipient.
